# EUROPÄISCHE PATENTSCHRIFT

(11) **EP 3 270 309 B1**
(45) Veröffentlichungstag und Bekanntmachung des Hinweises auf die Patenterteilung: **02.09.2020**
(21) Anmeldenummer: 17181266.2
(22) Anmeldetag: 13.07.2017
(51) Int. Cl.: G16H 40/63

(54) **ANZEIGEVORRICHTUNG FÜR EIN MEDIZINISCHES GERÄT**
DISPLAY DEVICE FOR A MEDICAL DEVICE
DISPOSITIF D'AFFICHAGE POUR UN APPAREIL MÉDICAL

(30) Priorität: 13.07.2016 DE 102016112886
(43) Veröffentlichungstag der Anmeldung: 17.01.2018
(73) Patentinhaber: B. Braun Avitum AG, 34212 Melsungen (DE)
(72) Erfinder: PETERS, Meike, 34212 Melsungen (DE); BRÖGGER, Sebastian, 34593 Knüllwald (DE); BRÖKER, Björn, 34355 Staufenberg (DE); STENZEL, Bruno, 34346 Hann. Münden (DE)
(74) Vertreter: Winter, Brandl, Fürniss, Hübner, Röss, Kaiser, Polte - Partnerschaft mbB

(56) Entgegenhaltungen:
- EP-A2- 1 101 502
- US-A1- 2015 070 319
- US-A1- 2015 187 196
- US-A1- 2016 015 330

## Beschreibung

Die Erfindung betrifft eine Anzeigevorrichtung für eine extrakorporale Blutbehandlungsmaschine/Dialysemaschine, und bezieht sich insbesondere auf einen bedienbaren und/oder zur Informationsdarstellung angepassten Bildschirm oder Monitor nach dem Prinzip einer Blickfelddarstellungseinrichtung oder eines HUD (Head Up Display).

Auf beispielsweise dem Gebiet der Vorrichtungen zur Durchführung einer extrakorporalen Blutbehandlung, wie beispielsweise einer Dialysemaschine, werden Bedien- und Anzeigegeräte in der Regel als Monitor mit eigenem Gehäuse oder in das Gerätegehäuse integriert ausgeführt. Aufgrund der üblicherweise vorgesehenen Bildschirmgröße, beispielsweise 38,1 cm entsprechend 15", sind aufwendige Maßnahmen zur Abdichtung und für hinreichenden EMV-Schutz notwendig. Außerdem entstehen an einem Übergang zwischen Monitorgehäuse und Bildschirm häufig Störkonturen, welche Reinigungsvorgänge erschweren. Bei Systemen mit einem berührungsempfindlichen Bedienfeld sind darüber hinaus hohe Anforderungen an die Ebenheit des Gehäuses im Bereich der berührungsempfindlichen Fläche zu erfüllen. Zudem ergeben sich häufig Parallaxenfehler aufgrund des Abstandes zwischen Monitor und berührungsempfindlicher Fläche und der Position des Bedieners oder Benutzers relativ zur Frontoberfläche.

US 2015/0187196 A1 offenbart einen Brutkasten für Frühgeborene, welche diverse Anzeige- und Projektionsflächen zum Anzeigen von verschiedenen Nachrichten und Alarmen aufweist, wie eine transparente Wand des Brutkastens oder eine an einem Arm verschwenkbar angeordnete Projektionsfläche.

Der Erfindung liegt als eine Aufgabe zugrunde, eine Anzeigevorrichtung für eine extrakorporale Blutbehandlungsmaschine, welche nachfolgend auch als medizinsiches Gerät bezeichnet wird, bereitzustellen, welche die vorstehenden Nachteile überwindet und ferner eine Anzeige von Betriebszuständen, eine interaktive Bedienung und/oder die Darstellung von Alarmanzeigen sicherstellt.

Der Erfindung liegt die allgemeine Idee zugrunde, in oder an einer Vorrichtung zur Durchführung einer extrakorporalen Blutbehandlung eine transluzente Projektionsfläche anzuordnen, die durch einen Projektor angestrahlt wird. Eine bevorzugte Projektionsrichtung ist eine Richtung, in welcher kein Schattenwurf des Bedieners oder Benutzers erfolgt. Die Projektionsfläche kann aus einer Glasscheibe, einem Kunststoff oder einem anderen Werkstoff mit entsprechender Opazität bestehen und freistehend und/oder verstellbar, beispielsweise neigbar, drehbar und/oder höhenverstellbar, sein. Auf der Projektionsfläche kann zumindest ein Interaktionselement vorgesehen sein, das mit einer kapazitiven oder resistiven Sensorik (berührungsempfindliche Schicht bzw. Touchscreen) ausgestattet sein kann. Eine Auswertung einer Interaktion des Bedieners oder Benutzers kann über eine Kamera oder eine andere optische bzw. kapazitive Sensorik erfolgen. Beispielsweise kann eine Konfiguration für eine 2D-Gestik- /2D-Positionserkennung auf der Projektionsebene, z.B. eines Fingers des Bedieners oder Benutzers, und/oder eine Konfiguration für eine 3D-Gestikerkennung implementiert sein. Ferner können auf bzw. an der Projektionsfläche Bedienelemente, beispielsweise Tastenanordnungen oder Hardkeys, integriert sein. Vermittels der Transluzenz der Projektionsfläche ist eine Betriebszustandsanzeige des medizinischen Geräts rundum sichtbar. In einem Alarmfall kann die Fläche der Betriebszustandsanzeige größer konfiguriert sein als in anderen Betriebsfällen und beispielsweise einen Informationsanzeigebereich rahmenförmig umgeben oder das gesamte Informationsanzeigefeld bzw. die gesamte Projektionsfläche ausleuchten und/oder eine Informationsanzeige überlagern, wobei durch Reflexionen auf dem Gerät die Signalwirkung verstärkt werden kann. Für eine Zweikanaligkeit einer Interaktions- oder Eingabeauswertung kann diese durch Auswertung mittels zumindest zweier Positionserkennungssysteme, beispielsweise einem auf Ultraschall und Berührungsempfindlichkeit basierenden Positionserkennungssystem und einem auf einem 3D-Kamerasystem und einer Infrarotabtastung mittels beispielsweise einem Infrarotschrankengitter basierenden Positionserkennungssystem, realisiert sein. Entlang der vorstehenden allgemeinen Idee entfallen aufgrund der freistehenden Projektionsfläche sowohl EMV-anfällige Elektronik im Bereich der Anzeigevorrichtung (Monitor) als auch EMV-Emissionen, kann eine zu fordernde Tropfwasserbeständigkeit, beispielsweise nach IP21, einfacher sichergestellt werden, und ist aufgrund der Gehäuselosigkeit und dadurch fehlender Ecken und/oder Kanten die Reinigung erleichtert und eine Beständigkeit gegenüber beispielsweise Desinfektionsmitteln erhöht. Aufgrund des Entfalls der bei bekannten Anordnungen vorgesehenen Dünnfilmtransistoranzeigen ist ein klein dimensionierbarer Projektor verwendbar, und ist damit einhergehend eine Anzeigebildgröße leicht skalierbar, sowie können separate Betriebszustandsanzeigen entfallen. Insgesamt sind darüber hinaus die Herstellungskosten geringer, und ist in Verbindung mit einer abklappbaren Projektionsfläche oder einer Montage derselben am Aufstellort durch einen Techniker ein Verpackungsvolumen des Geräts geringer, woraus wiederum geringere Transportkosten resultieren.

Im Einzelnen wird die Aufgabe gelöst durch eine extrakorporale Blutbehandlungsmaschine mit einer Anzeigevorrichtung mit einer Projektionsfläche, die an dem medizinischen Gerät montiert und dazu konfiguriert ist, einen vorbestimmten Anzeigeinhalt für einen Benutzer des medizinischen Geräts auf einer Vorderseite der Projektionsfläche/-platte sichtbar darzustellen, und einer (zur Projektionsfläche parallel beabstandeten, lichtemittierenden) Projektionsvorrichtung, die an dem medizinischen Gerät montiert und dazu konfiguriert ist, den vorbestimmten Anzeigeinhalt von einer Rückseite der Projektionsfläche her auf die Projektionsfläche zu projizieren. Dabei ist ein vorbestimmter Abschnitt der Projektionsfläche zur Anzeige zumindest eines vorbestimmten Betriebszustands des medizinischen Geräts vorgesehen. Ferner ist in zumindest einem ersten und einem zweiten Betriebszustand ein vorbestimmter erster und/oder zweiter Abschnitt der Projektionsfläche ansteuerbar dazu konfiguriert, den ersten und den zweiten Betriebszustand verschiedenfarbig anzuzeigen. Dabei ist die Projektionsfläche mittels einer anlenkenden Vorrichtung, welche eine scharnierartige Gelenkanordnung ist, an einem Oberflächenabschnitt der extrakorporalen Blutbehandlungsmaschine an einer tischförmigen Ausformung oder Erhebung angelenkt und/oder in einer gegenüber der extrakorporalen Blutbehandlungsmaschine in zumindest Höhenrichtung festgelegten Rahmenanordnung geführt und innerhalb dieser verschiebbar. Die anlenkende Vorrichtung und/oder die Rahmenanordnung sind insgesamt derart höhenbeweglich konfiguriert, dass die Projektionsfläche zumindest teilweise in eine Ausnehmung der extrakorporalen Blutbehandlungsmaschine einschiebbar ist.

Bevorzugt ist die Projektionsfläche eine aus einem opaken Werkstoff bestehende, transluzente Projektionsfläche und kann aus beispielsweise Glas oder Kunststoff bestehen. Eine Projektionsfläche aus Glas weist vorteilhaft eine verbesserte Kratzfestigkeit und Widerstandsfähigkeit gegenüber im Umfeld des Geräts vorkommenden Substanzen (Reinigungs- und Desinfektionsmitteln) auf. Eine Projektionsfläche aus Kunststoff stellt vorteilhaft eine erhöhte Bruchfestigkeit bereit und kann leichter ausgebildet sein.

Bevorzugt ist die Projektionsvorrichtung neigbar, drehbar und/oder höhenverstellbar am medizinischen Gerät/dessen Gehäuse angeordnet. Falls die Projektionsvorrichtung verstellbar ausgeführt ist, kann sie derart an dem medizinischen Gerät/dessen Gehäuse angelenkt/montiert sein, dass sie zur Aufrechterhaltung einer Projektion des vorbestimmten Anzeigeinhalts einer Neigung, Drehung und/oder Höhenverstellung der Projektionsfläche nachführbar ist.

Bevorzugt beinhaltet die Anzeigevorrichtung ferner ein Interaktionselement, das auf der Projektionsfläche angeordnet ist und dazu konfiguriert ist, eine Interaktion des Benutzers berührungsempfindlich, beispielsweise kapazitiv oder resistiv, zu erfassen. Eine so erfasste Interaktion kann vorteilhaft auf einfache Weise einer nachgeordneten Steuereinheit oder Signalverarbeitungseinheit für eine vorbestimmte Weiterverarbeitung zugeleitet werden.

Alternativ bevorzugt kann die Anzeigevorrichtung ferner eine bildgebende Einrichtung und/oder optische Sensoreinrichtung beinhalten, die dazu konfiguriert ist, eine Interaktion des Benutzers berührungslos zu erfassen. Vorteilhaft kann eine derartige Erfassung über ein integriertes, beispielsweise in die Projektionsvorrichtung integriertes, oder separat angeordnetes bildgebendes System oder Kamerasystem erfolgen.

Bevorzugt ist die Anzeigevorrichtung dazu konfiguriert, eine 2D- und/oder 3D- Gestik- und/oder Positionserkennung auf einer Projektionsebene der Anzeigevorrichtung oder der Projektionsvorrichtung durchzuführen. Auf diese Weise kann der Bediener oder Benutzer, dem vermittels der Projektion eine Art virtuelle Bedienoberfläche präsentiert wird, bei geeigneter Annäherung an die virtuelle Bedienoberfläche mit dem medizinischen Gerät interagieren, während an entfernteren Positionen keine Reaktion des medizinischen Geräts ausgelöst wird.

Bevorzugt kann alternativ oder zusätzlich ein durch den Benutzer manuell betätigbares Bedienelement in die Projektionsfläche integriert sein. Vorteilhaft kann das zumindest eine Bedienelement funktionell festverdrahtet konfiguriert sein, d.h. es kann ihm eine vorbestimmte Funktion dauerhaft zugewiesen sein. Alternativ vorteilhaft kann das zumindest eine Bedienelement funktionell frei konfigurierbar sein, d.h. es kann ihm eine vorbestimmte Funktion, beispielsweise aus einem Satz vordefinierter Funktionen, über einen Geräteeinstellbereich oder dergleichen veränderlich oder temporär zuweisbar sein. Das Bedienelement kann eine Bedientaste, ein Drehsteller, ein Dreh-/Drück-Steller oder dergleichen sein.

Weiter bevorzugt kann dabei in einem dritten Betriebszustand ein vorbestimmter dritter Abschnitt der Projektionsfläche ansteuerbar konfiguriert sein, den dritten Betriebszustand gegenüber dem ersten und dem zweiten Betriebszustand andersfarbig anzuzeigen, wobei der vorbestimmte dritte Abschnitt größer ist als der vorbestimmte erste Abschnitt und der vorbestimmte zweite Abschnitt. Vorteilhaft ist in einer solchen Konfiguration der vorbestimmte erste Abschnitt eine erste Teilfläche der Projektionsfläche und ist der vorbestimmte zweite Abschnitt eine zweite Teilfläche der Projektionsfläche, ist der erste Betriebszustand ein Normalbetriebszustand des medizinischen Geräts und ist der zweite Betriebszustand ein Meldebetriebszustand des medizinischen Geräts, und ist der vorbestimmte dritte Abschnitt eine rahmende Teilfläche oder Gesamtfläche der Projektionsfläche und ist der dritte Betriebszustand ein Alarmzustand des medizinischen Geräts, wobei die Anzeige der Betriebszustände der Anzeige des vorbestimmten Anzeigeinhalts überlagerbar sein kann. Vorteilhaft können als verschiedene Farben zur Anzeige jeweils unterschiedlicher Betriebszustände Ampelfarben Grün (für beispielsweise einen ordnungsgemäßen oder regelmäßigen Betriebszustand), Gelb (für beispielsweise einen Betriebszustand, in dem das medizinische Gerät eine Meldung oder dergleichen ausgibt und erhöhte Aufmerksamkeit des Bedieners oder Benutzers anfordert) und Rot (für beispielsweise einen Alarmbetriebszustand wie beispielsweise bei Auftreten einer Störung oder einer kritischen Situation während eines Behandlungsverlaufs) herangezogen werden. Weiter vorteilhaft können Position und Größe der einzelnen Abschnitte abhängig von einem Betriebszustand, einem Verfahrensstand und/oder einer Dringlichkeit veränderlich sein, wodurch die von dem Bediener oder Benutzer angeforderte Aufmerksamkeit steuerbar wird. Beispielsweise kann ein grün ausgeleuchteter Abschnitt rein bestätigend in unauffälliger Größe und an unauffälliger Position bereitgestellt werden, kann ein gelb ausgeleuchteter Abschnitt dringlichkeitsabhängig großen- und/oder positionsvariabel bereitgestellt werden, und kann ein sofortige Maßnahmen unbedingt anfordernder, rot ausgeleuchteter Abschnitt rahmenförmig oder ganzflächig überlagernd bereitgestellt werden. Darüber hinaus können Leuchtintensität und/oder Frequenz der Bereitstellung parameterabhängig oder dringlichkeitsabhängig veränderlich sein.

Bevorzugt ist bei der Anzeigevorrichtung eine zumindest zweikanalige Eingabeauswertung einer Interaktion des Benutzers mittels zumindest einem ersten und einem zweiten Positionserkennungssystem vorgesehen. Vorteilhaft wird dadurch die in manchen medizinischen Anwendungsfällen erforderliche Zweikanaligkeit der Auswertung bereitgestellt. Vorteilhaft kann dabei das erste Positionserkennungssystem eine Ultraschalleinrichtung und eine berührungsempfindliche Einrichtung beinhalten, und kann das zweite Positionserkennungssystem eine 3D-Kamera und eine Infrarotabtastung beinhalten. Das erste und/oder das zweite Positionserkennungssystem können in beispielsweise die Projektionsvorrichtung integriert oder andernorts an geeigneter Stelle bezüglich des medizinischen Geräts angeordnet sein.

Bevorzugt sind bei der Anzeigevorrichtung die Projektionsfläche und die Projektionsvorrichtung als HUD-Einheit (Head Up Display-Einheit) zur Blickfelddarstellung des Anzeigeinhalts konfiguriert.

Die vorstehende Kurzbeschreibung ist lediglich illustrativ und in keiner Weise beschränkend. Die Erfindung wird nachstehend anhand eines bevorzugten Ausführungsbeispiels unter Bezugnahme auf die beigefügte Zeichnung näher beschrieben. Soweit nicht anders angegeben, bezeichnen in der Zeichnung gleiche Bezugszeichen jeweils gleiche Komponenten, die nicht redundant beschrieben werden. Es zeigen:
Fig. 1 eine schematische Darstellung eines medizinischen Geräts mit einer Anzeigevorrichtung und einer Projektionsvorrichtung gemäß einem Ausführungsbeispiel;
Fig. 2 in schematischer Darstellung das medizinische Gerät nach Fig. 1 mit aktivierter Projektion eines Anzeigeinhalts auf eine Projektionsfläche der Anzeigevorrichtung; und
Fig. 3A bis 3C jeweils schematische Darstellungen der Projektionsfläche mit beispielhaft als Tasten oder Schalter ausgeführten Bedien- oder Interaktionselementen (Fig. 3A), einer Betriebszustandsanzeige für einen Normal- oder Meldebetriebszustand des medizinischen Geräts (Fig. 3B) und einer Betriebszustandsanzeige für einen Alarmbetriebszustand des medizinischen Geräts (Fig. 3C).

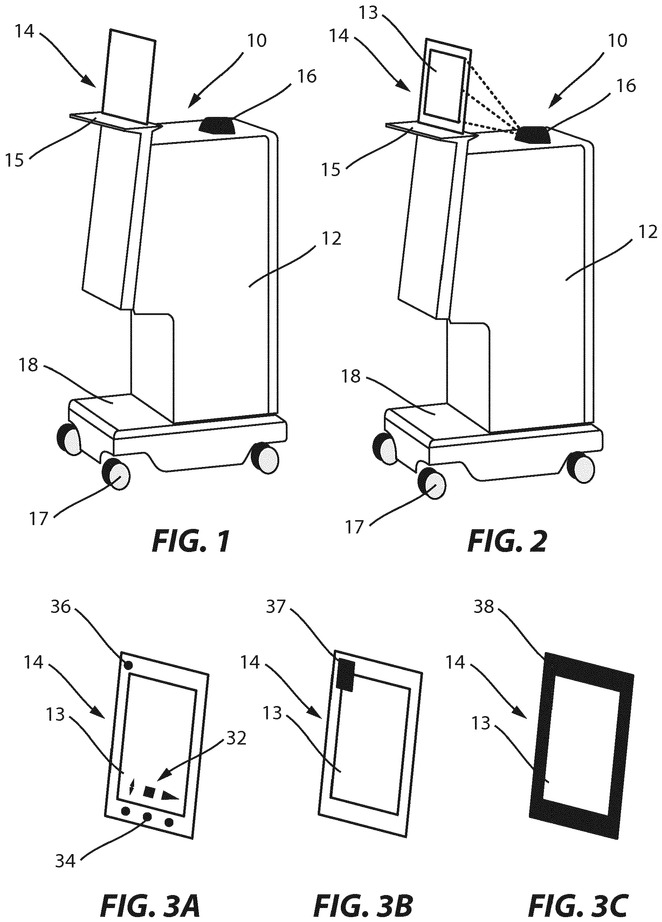

Wie in Fig. 1 und Fig. 2 vereinfacht und schematisch dargestellt, beinhaltet ein medizinisches Gerät 10, beispielsweise ein Gerät zur extrakorporalen Blutbehandlung wie beispielsweise eine Dialysemaschine, einen Korpus 12, in bzw. an welchem innenseitig und außenseitig systembedingt erforderliche Komponenten aufgenommen sind. Die systembedingt erforderlichen Komponenten eines jeweils tatsächlichen medizinischen Geräts 10 sind an sich bekannt und werden insoweit weder explizit dargestellt noch näher beschrieben. Das medizinische Gerät 10 kann über Rollen 17 verfahrbar auf einem Sockel 18 angeordnet sein.

Eine Projektionsfläche 14 und eine Projektor- oder Projektionsvorrichtung 16, die in einem vorbestimmten Abstand zu der Projektionsfläche 14 angeordnet ist, bilden in diesem Ausführungsbeispiel eine Anzeigevorrichtung für das medizinische Gerät 10, bei der die Projektionsfläche 14 an dem medizinischen Gerät 10 angeordnet und dazu konfiguriert ist, einen vorbestimmten Anzeigeinhalt 13 für einen Benutzer des medizinischen Geräts 10 sichtbar darzustellen, und die Projektionsvorrichtung 16 ebenfalls an dem medizinischen Gerät 10 angeordnet und dazu konfiguriert ist, den vorbestimmten Anzeigeinhalt 13 von einer Rückseite der Projektionsfläche 14 her auf die Projektionsfläche bzw. eine Projektionsebene, die vorzugsweise auf bzw. an der Projektionsfläche 14 liegt, zu projizieren.

Die Anzeigevorrichtung für das medizinische Gerät 10 mit Projektionsfläche 14 und Projektionsvorrichtung 16 arbeitet somit nach dem Prinzip einer Informationsanzeigeeinrichtung mit Blickfelddarstellung, die begrifflich auch als Head Up Display (HUD) bezeichnet wird.

Genauer kann die Anzeigevorrichtung in dem vorliegenden Ausführungsbeispiel beispielsweise eine Informationsbeschaffungseinheit und eine Bildanzeigesteuereinheit beinhalten. Die Bildanzeigesteuereinheit kann eine Lichtquelle und ein bewegliches reflektives Element beinhalten. Die Lichtquelle ist dazu angeordnet, einen Lichtstrahl auszugeben. Die Anzeigevorrichtung bzw. deren Projektionsvorrichtung 16 ist dazu konfiguriert, den Lichtstrahl auf die Projektionsfläche 14, d.h. fokussiert auf eine in der Projektionsfläche 14 liegende Ebene, zu projizieren, um die Anzeigeinformation anzuzeigen. Die Informationsbeschaffungseinheit kann mit einem oder mehreren Sensoren und/oder einer Steuereinheit gekoppelt sein und beispielsweise von dem einen oder den mehreren Sensoren erfasste Messwerte oder Daten und/oder von der Steuereinheit geräteintern generierte, beispielsweise auf einen Behandlungsverlauf oder Gerätezustand bezogene Parameter und/oder Information holen, die geholten Daten und/oder die geholte Information gemäß einer vorbestimmten Verarbeitung verarbeiten und die verarbeitete Information in beispielsweise Bildform und/oder Textform, ohne darauf beschränkt zu sein, an die Bildanzeigesteuereinheit ausgeben. Die Informationsbeschaffungseinheit kann darüber hinaus Anzeigesteuersignale auf der Grundlage der Daten für die Bildanzeigesteuereinheit bereitstellen. Die Bildanzeigesteuereinheit kann die verarbeitete Information empfangen und sie auf die Projektionsfläche 14 projizieren, und darüber hinaus die Anzeigesteuersignale empfangen, um vermittels beispielsweise des beweglichen reflektiven Elements eine Position der Anzeigeinformation auf der Projektionsfläche 14 festzulegen bzw. einzustellen.

In diesem Ausführungsbeispiel ist die Projektionsfläche 14 eine aus einem opaken Werkstoff bestehende, transluzente Projektionsfläche, beispielsweise nach Art einer Streuscheibe oder dergleichen. Beispielsweise kann die Projektionsfläche 14 aus einem ebenflächig oder gekrümmt geformten und starren, zumindest aber verwindungssteifen, Glas oder Kunststoff vorbestimmter Größe und Dicke bestehen, in welche die Anzeigeinformationsprojektion der Projektionsvorrichtung 16 größenvariabel und auf eine Ebene innerhalb der Projektionsfläche 14 so einpassbar oder aufstrahlbar ist, dass der Anzeigeinhalt 13 fokussiert und für einen Bediener oder Benutzer lesbar dargestellt wird.

Ferner sind in diesem Ausführungsbeispiel die Projektionsfläche 14 und gegebenenfalls auch die Projektionsvorrichtung 16 neigbar, drehbar und/oder höhenverstellbar angeordnet.

Beispielsweise kann die Projektionsfläche 14 mittels einer scharnierartigen Gelenkanordnung an einem Oberflächenabschnitt des medizinischen Geräts 10, z.B. an einer tischförmigen Ausformung oder Erhebung 15, verschwenkbar, etwa in einer Richtung nach vorne und/oder hinten bezüglich einer Stirnseite des medizinischen Geräts 10 klappbar oder neigbar, und/oder um eine Höhenrichtung drehbar, angelenkt sein. Ein Dreh- und/oder Neigungswinkelbereich kann beispielsweise in einem Bereich vorgesehen sein, der einerseits eine Ablesbarkeit des Anzeigeinhalts 13 durch eine vor dem medizinischen Gerät 10 stehende Person noch ermöglicht und andererseits ein Umklappen in eine schützende Liege- oder Ruhestellung, beispielsweise in eine weich ummantelnde Ausnehmung an der tischförmigen Ausformung oder Erhebung, erlaubt.

Weiter beispielsweise kann die Projektionsfläche 14 durch etwa eine Höhenverstellbarkeit einer die Projektionsfläche 14 an dem medizinischen Gerät 10 anlenkenden Anordnung in einer vorbestimmt geneigten Position in einer Richtung nach oben und/oder unten bezüglich einer oberen Oberfläche des medizinischen Geräts 10 in ihrer absoluten Höhe über Grund einstellbar sein. Alternativ kann beispielsweise die Projektionsfläche 14 in einer gegenüber dem medizinischen Gerät 10 in zumindest Höhenrichtung festgelegten Rahmenanordnung geführt und innerhalb dieser verschiebbar sein. In einer Modifikation können die vorstehend beispielsweise genannte anlenkende Vorrichtung und/oder eine Rahmenanordnung insgesamt derart höhenbeweglich konfiguriert sein, dass die Projektionsfläche 14 zumindest teilweise in eine Ausnehmung des medizinischen Geräts 10 versenkbar bzw. einschiebbar ist.

Gegebenenfalls vorzusehende elektrische Leiterverbindungen können dazu in der anlenkenden Vorrichtung und/oder der Rahmenanordnung verdeckt und in entsprechender Überlänge geführt sein. In einer weiteren Modifikation können solche elektrischen Leiterverbindungen über eine trennbare Steckkontaktanordnung geführt und die Projektionsfläche 14 als Ganzes abnehmbar ausgebildet sein, so dass für den Fall eines Bruchs der Projektionsfläche 14 ein einfacher Austausch möglich und in beispielsweise einem Nichtbetriebszustand des medizinischen Geräts 10 die Projektionsfläche 14 an einem diese sicher schützenden Ort aufbewahrbar ist.

In diesem Ausführungsbeispiel kann ferner ein Bedien- oder Interaktionselement 32 (vgl. Fig. 3A) auf der Projektionsfläche 14 angeordnet und dazu konfiguriert sein, eine Interaktion des Benutzers berührungsempfindlich, beispielsweise kapazitiv oder resistiv, zu erfassen. Das Interaktionselement 32 kann beispielsweise ein Softwareschalter sein, der vermittels der Projektionsvorrichtung 16 softwaregesteuert in den Anzeigeinhalt 13 einprojiziert und dessen Zustand oder Betätigung über eine vorzugsweise Mehrpunkt- bzw. Multitouch-fähige berührungsempfindliche (Touch)-Schicht an der Projektionsfläche 14 erfasst wird, und der z.B. ein Verändern eines Parameters des medizinischen Geräts erlaubt oder dem Bediener oder Benutzer Steuerungsfunktionen des medizinischen Geräts 10, wie beispielsweise Start, Stopp und Pausieren eines Betriebsablaufs, eine Aufzeichnung eines zeitlichen Ablaufs und dergleichen, zugänglich macht.

Alternativ oder zusätzlich kann eine bildgebende Einrichtung und/oder optische Sensoreinrichtung angeordnet sein, die dazu konfiguriert ist, eine Interaktion des Benutzers berührungslos zu erfassen. In diesem Fall kann eine berührungsempfindliche Schicht entfallen und eine Interaktion beispielsweise durch eine an sich bekannte Kameraanordnung als bildaufnehmende und/oder bildgebende Einrichtung die Position eines Fingers des Bedieners oder Benutzers erfassen und zur Eingabeauswertung an eine nachgeschaltete Verarbeitungs- oder Steuereinrichtung, beispielsweise den Mikrocontroller, leiten.

In diesem Ausführungsbeispiel ist es mit den vorgenannten Erfassungseinrichtungen zur Erfassung und Auswertung einer Interaktion oder Eingabe eines Bedieners oder Benutzers möglich, eine 2D- und/oder 3D-Gestik- und/oder Positionserkennung auf einer Projektionsebene der Projektionsvorrichtung durchzuführen. Insbesondere kann dabei eine zumindest zweikanalige Eingabeauswertung einer Interaktion des Benutzers mittels zumindest einem ersten und einem zweiten Positionserkennungssystem vorgesehen sein, wobei das erste Positionserkennungssystem beispielsweise eine Ultraschalleinrichtung und eine berührungsempfindliche Einrichtung beinhalten kann und das zweite Positionserkennungssystem beispielsweise eine 3D-Kamera und eine Infrarotabtastung beinhalten kann.

Weiter alternativ oder zusätzlich kann an der Projektionsfläche 14 und/oder an der Projektionsvorrichtung 16 zumindest eine durch den Benutzer manuell betätigbare Bedientaste 34 integriert sein (vgl. Fig. 3A). Die zumindest eine Bedientaste 34 kann in Form beispielsweise eines Hardwareschalters (Hardkey) vorgesehen sein, der funktionell festverdrahtet konfiguriert ist, d.h. gleichbleibend eine vorbestimmte Funktion an beispielsweise dem medizinischen Gerät 10 oder der Projektionsvorrichtung 16 auslöst, oder in vergleichbarem Sinne funktionell frei konfigurierbar ist, beispielsweise durch eine programm- oder betriebsablaufabhängige Belegung durch Software nach Art einer Funktionstaste.

Ferner ist in diesem Ausführungsbeispiel ein vorbestimmter Abschnitt der Projektionsfläche 14 zur Anzeige eines vorbestimmten Betriebszustands des medizinischen Geräts 10 vorgesehen, der beispielsweise dazu konfiguriert ist, auf der Projektionsfläche 14 verschiedene Betriebszustände verschiedenfarbig hervorzuheben und dadurch entsprechende Aufmerksamkeit des Bedieners oder Benutzers zu erregen.

In einer Modifikation kann die Projektionsvorrichtung 16 dazu einen Betriebszustandsanzeigeabschnitt (nicht gezeigt) beinhalten, der eine zweite oder sekundäre (Sub-)Projektionsvorrichtung bilden kann, separat von oder zusätzlich zu einem für die Projektion der Anzeigeinformation zuständigen Anzeigeinformationsprojektionsabschnitt bereitgestellt sein kann, und angesteuert durch beispielsweise einen Mikrocontroller dazu konfiguriert sein kann, in zumindest einem Bereich der Projektionsfläche 14 farblich erkennbar abgesetzt von der Anzeigeinformation ein vorzugsweise flächiges Element anzuzeigen, einzublenden oder der Anzeigeinformation zu überlagern, wobei eine Leuchtintensität, eine Aufleuchtfrequenz (blinkende Darstellung), eine Größe, eine Form und/oder eine Position des flächigen Elements in Abhängigkeit von einem zugeordneten Betriebszustand und/oder einer Dringlichkeit der dadurch zu übermittelnden Betriebszustandsinformation veränderlich sein können (vgl. Fig. 3A bis Fig. 3C).

Beispielsweise kann eine Konfiguration unter Verwendung sogenannter Ampelfarben derart sein, dass eine erste Betriebszustandsanzeige 36, die über einen störungsfreien und regelmäßigen Betriebszustand (Normalbetriebszustand) informiert, in grüner Farbe, einer ersten Größe und/oder Form und an einer ersten Position, die eine eher unauffällige Position innerhalb der Projektionsfläche sein kann, dargestellt wird, eine zweite Betriebszustandsanzeige 37, die über einen zwar unkritischen, aber zumindest Kenntnisnahme und/oder möglicherweise Interaktion durch den Bediener oder Benutzer erfordernden zweiten Betriebszustand (Meldebetriebszustand) oder Vorgang während eines Behandlungsablaufs informiert, in gelber Farbe, einer zweiten Größe, die gleich oder größer ist als die erste Größe, und/oder zweiten Form, die von der ersten Form unterscheidbar ist, und an einer zweiten Position, die eine gegenüber der auffälligere Position innerhalb der Projektionsfläche sein kann, dargestellt wird, und eine dritte Betriebszustandsanzeige 38, die über einen kritischen und eine wenigstens zeitnahe Interaktion durch den Bediener oder Benutzer erfordernden dritten Betriebszustand (Alarmzustand) oder Vorgang während eines Behandlungsablaufs informiert, in roter Farbe, einer zweiten Größe, die größer ist als die erste und die zweite Größe, und/oder einer zweiten Form, die von der ersten und der zweiten Form unterscheidbar ist, und an einer zweiten Position, die eine gegenüber der auffälligere Position innerhalb der Projektionsfläche sein kann, dargestellt wird. In diesem Ausführungsbeispiel wird beispielsweise die erste Betriebszustandsanzeige 36 durch einen runden grünen Punkt dargestellt (Fig. 3A), die zweite Betriebszustandsanzeige 37 durch ein gelbes Rechteck größer als der runde grüne Punkt dargestellt, und die dritte Betriebszustandsanzeige 38 durch die Projektionsfläche 14 umgebenden roten Rahmen dargestellt.

In anderen Worten ist in diesem Ausführungsbeispiel in zumindest dem ersten und dem zweiten Betriebszustand ein vorbestimmter erster Abschnitt der Projektionsfläche 14 ansteuerbar dazu konfiguriert, den ersten und den zweiten Betriebszustand verschiedenfarbig anzuzeigen, und in einem dritten Betriebszustand ein vorbestimmter zweiter Abschnitt der Projektionsfläche 14 ansteuerbar dazu konfiguriert, den dritten Betriebszustand gegenüber dem ersten und dem zweiten Betriebszustand andersfarbig anzuzeigen, wobei vorzugsweise der vorbestimmte zweite Abschnitt größer ist als der vorbestimmte erste Abschnitt, wobei der vorbestimmte erste Abschnitt eine erste Teilfläche der Projektionsfläche 14 sein kann, der erste Betriebszustand ein Normalbetriebszustand des medizinischen Geräts 10 sein kann und der zweite Betriebszustand ein Meldebetriebszustand des medizinischen Geräts 10 sein kann, und der vorbestimmte zweite Abschnitt eine rahmende Teilfläche oder eine Gesamtfläche der Projektionsfläche 14 sein kann und der dritte Betriebszustand ein Alarmzustand des medizinischen Geräts 10 sein kann, wobei die Anzeige der Betriebszustände der Anzeige des vorbestimmten Anzeigeinhalts 13 überlagerbar ist.

In einer weiteren Modifikation dieses Ausführungsbeispiels können berührungsempfindliche Interaktionselemente 32 (vgl. Fig. 3A) derart mit dem ersten, dem zweiten und/oder dem dritten Abschnitt kombiniert sein, dass beispielsweise der Betriebszustandsanzeigeabschnitt der Projektionsvorrichtung oder eine andere dazu angeordnete Steuereinrichtung des medizinischen Geräts 10 bei Erzeugung oder Durchführung einer Betriebszustandsanzeige gleichzeitig ein berührungsempfindliches Interaktionselement 32 an entsprechender Stelle auf der Projektionsfläche 14 einrichtet und für eine Benutzereingabe aktiviert.

Auf diese Weise kann ein Bediener oder Benutzer unmittelbar auf die Betriebszustandsanzeige reagieren, beispielsweise eine Hinweis- oder Warnmeldung (gelb) zurücksetzen oder in einem Alarmzustand (rot) eine Notabschaltung auslösen. Im letztgenannten Fall kann es dann genügen, für einen Nothalt oder einen Übergang auf einen sicheren Betriebsablauf des medizinischen Geräts 10 irgendeine Stelle innerhalb des (roten) dritten Abschnitts der dritten Betriebszustandsanzeige zu berühren, wodurch in diesem Fall eine Reaktionszeit eines Bedieners oder Benutzers vorteilhaft verkürzt werden kann.

Alternativ oder zusätzlich können bestimmte Gesten vordefiniert sein, die bei Ausgabe einer zugeordneten Betriebszustandsanzeige aktivierbar sind und nach Aktivierung durch die 2D- und/oder 3D-Positions- und/oder -Gestenerkennung als Reaktion eines Bedieners oder Benutzers auf einen eingetretenen Betriebszustand erkannt werden. In diesem Fall kann beispielsweise eine vorbestimmte Handbewegung oder Stellung zweier Hände zueinander in einem Alarmzustand den Nothalt oder den Übergang auf den sicheren Betriebsablauf des medizinischen Geräts 10 auslösen, während in einem Nichtalarmzustand dieselbe Handbewegung oder Händestellung keine Reaktion des medizinischen Geräts 10 bewirkt

Insgesamt sind somit in diesem Ausführungsbeispiel die Projektionsfläche 14 und die Projektionsvorrichtung 16 wie vorstehend beschrieben als eine HUD-Einheit zur Blickfelddarstellung einer auf die Projektionsfläche 14 zu projizierenden Anzeigeinformation (Anzeigeinhalt 13) konfiguriert, und können als solche (passiv und/oder interaktiv) funktionelle Teile oder Komponenten einer Vorrichtung zur Durchführung einer extrakorporalen Blutbehandlung, wie beispielsweise einer Dialysemaschine, bilden.

Wie vorstehend beschrieben wurde, beinhaltet eine Anzeigevorrichtung für ein medizinisches Gerät 10 eine Projektionsfläche 14, die an dem medizinischen Gerät 10 angeordnet und dazu konfiguriert ist, einen vorbestimmten Anzeigeinhalt 13 für einen Benutzer des medizinischen Geräts 10 sichtbar darzustellen, und eine Projektionsvorrichtung 16, die an dem medizinischen Gerät 10 angeordnet und dazu konfiguriert ist, den vorbestimmten Anzeigeinhalt 13 von einer Rückseite der Projektionsfläche 14 her auf die Projektionsfläche 14 zu projizieren. Die Projektionsfläche 14 und die Projektionsvorrichtung 16 sind als HUD-Einheit zur Blickfelddarstellung des Anzeigeinhalts 13 konfiguriert, und das medizinische Gerät 10 kann eine Vorrichtung sein zur Durchführung einer extrakorporalen Blutbehandlung, die eine solche Anzeigevorrichtung aufweist.

Die Erfindung wurde vorstehend anhand eines bevorzugten Ausführungsbeispiels beschrieben. Es versteht sich, dass Einzelheiten des beschriebenen bevorzugten Ausführungsbeispiels die Erfindung als solche nicht beschränken und sich für den Fachmann naheliegend verschiedenartige Änderungen, Modifikationen und/oder Äquivalente ergeben können, die als solche allesamt im Rahmen der durch die beigefügten Ansprüche definierten Schutzbereich der Erfindung liegen.

## Patentansprüche

1. Extrakorporale Blutbehandlungsmaschine mit einer Anzeigevorrichtung, beinhaltend:
eine Projektionsfläche oder -platte (14), die an der extrakorporalen Blutbehandlungsmaschine montiert und dazu konfiguriert ist, einen vorbestimmten Anzeigeinhalt (13) für einen Benutzer der extrakorporalen Blutbehandlungsmaschine auf einer Vorderseite der Projektionsfläche/-platte (14) sichtbar darzustellen; und
eine Projektionsvorrichtung (16), die an der extrakorporalen Blutbehandlungsmaschine montiert und dazu konfiguriert ist, den vorbestimmten Anzeigeinhalt (13) von einer Rückseite der Projektionsfläche (14) her auf die Projektionsfläche (16) zu projizieren,
**dadurch gekennzeichnet, dass**
ein vorbestimmter Abschnitt der Projektionsfläche (14) zur Anzeige zumindest eines vorbestimmten Betriebszustands der extrakorporalen Blutbehandlungsmaschine vorgesehen ist,
in zumindest einem ersten und einem zweiten Betriebszustand ein vorbestimmter erster und/oder zweiter Abschnitt (36, 37) der Projektionsfläche (14) ansteuerbar dazu konfiguriert ist, den ersten und den zweiten Betriebszustand verschiedenfarbig anzuzeigen,
die Projektionsfläche mittels einer anlenkenden Vorrichtung, welche eine scharnierartige Gelenkanordnung ist, an einem Oberflächenabschnitt der extrakorporalen Blutbehandlungsmaschine an einer tischförmigen Ausformung oder Erhebung angelenkt ist und/oder in einer gegenüber der extrakorporalen Blutbehandlungsmaschine in zumindest Höhenrichtung festgelegten Rahmenanordnung geführt und innerhalb dieser verschiebbar ist,
die anlenkende Vorrichtung und/oder die Rahmenanordnung insgesamt derart höhenbeweglich konfiguriert sind, dass die Projektionsfläche (14) zumindest teilweise in eine Ausnehmung der extrakorporalen Blutbehandlungsmaschine einschiebbar ist.

2. Extrakorporale Blutbehandlungsmaschine nach Anspruch 1, bei der die Projektionsfläche (14) eine aus einem semi-opaken Werkstoff bestehende, transluzente Projektionsfläche (14) ist.

3. Extrakorporale Blutbehandlungsmaschine nach einem der vorangehenden Ansprüche, ferner beinhaltend mindestens ein Interaktionselement (32, 34), das auf der Projektionsfläche (14) angeordnet ist und dazu konfiguriert ist, eine Interaktion des Benutzers berührungsempfindlich zu erfassen.

4. 6.Extrakorporale Blutbehandlungsmaschine nach einem der vorangehenden Ansprüche, ferner beinhaltend eine bildgebende Einrichtung und/oder optische Sensoreinrichtung, die dazu konfiguriert ist, eine Interaktion des Benutzers berührungslos zu erfassen.

5. Extrakorporale Blutbehandlungsmaschine nach einem der vorangehenden Ansprüche, die dazu konfiguriert ist, eine 2D- und/oder 3D- Gestik- und/oder Positionserkennung auf einer Projektionsebene durchzuführen.

6. Extrakorporale Blutbehandlungsmaschine nach einem der vorangehenden Ansprüche, bei der zumindest ein durch den Benutzer manuell betätigbares Bedienelement (34) in die Projektionsfläche (14) integriert ist.

7. Extrakorporale Blutbehandlungsmaschine nach Anspruch 1, bei der in einem dritten Betriebszustand ein vorbestimmter dritter Abschnitt (38) der Projektionsfläche (14) ansteuerbar konfiguriert ist, den dritten Betriebszustand gegenüber dem ersten und dem zweiten Betriebszustand andersfarbig anzuzeigen, wobei der vorbestimmte dritte Abschnitt (38) größer ist als der vorbestimmte erste Abschnitt (36) und der vorbestimmte zweite Abschnitt (37).

8. Extrakorporale Blutbehandlungsmaschine nach einem der Ansprüche 3 bis 5, bei der eine zumindest zweikanalige Eingabeauswertung einer Interaktion des Benutzers mittels zumindest einem ersten und einem zweiten Positionserkennungssystem vorgesehen ist.

## Claims

1. Extracorporeal blood treatment device having a display device, comprising:
a projection surface or plate (14) which is mounted to the extracorporeal blood treatment device and is configured to present a predetermined display content (13) in a way visible to a user of the extracorporeal blood treatment device on a front side of the projection surface/plate (14); and
a projection device (16) which is mounted on the extracorporeal blood treatment device and is configured to project the predetermined display content (13) from a rear side of the projection surface (14) onto the projection surface (16),
**characterized in that**
a predetermined portion of the projection surface (14) is provided for displaying at least one predetermined operating condition of the extracorporeal blood treatment device,
in at least a first and a second operating condition, a predetermined first and/or second portion (36, 37) of the projection surface (14) is controllably configured to display the first and second operating conditions in different colors,
the projection surface is articulated by means of an articulating device, being a hinge-type joint arrangement, at a surface portion of the extracorporeal blood treatment device at a table-shaped molding or elevation, and/or is guided within a frame arrangement fixed at least in the height direction vis-à-vis the extracorporeal blood treatment device and is slidable within it,
wherein the articulating device and/or the frame arrangement are overall configured to be movable in height such that the projection surface (14) can be inserted at least partially into a recess of the extracorporeal blood treatment device.

2. Extracorporeal blood treatment device according to claim 1, in which the projection surface (14) is a translucent projection surface (14) made from semi-opaque material.

3. Extracorporeal blood treatment device according to one of the preceding claims, further comprising at least one interactive element (32, 34) which is arranged on the projection surface (14) and is configured to detect an interaction of the user in a touch-sensitive manner.

4. Extracorporeal blood treatment device according to one of the preceding claims, further comprising an imaging means and/or optical sensor means which is configured to detect interaction of the user in a contactless manner.

5. Extracorporeal blood treatment device according to one of the preceding claims, which is configured to carry out 2D and/or 3D gesture and/or position detection on a projection plane.

6. Extracorporeal blood treatment device according to one of the preceding claims, in which at least one operating element (34) manually operable by the user is integrated in the projection surface (14).

7. Extracorporeal blood treatment device according to claim 1, in which in a third operating condition a predetermined third portion (38) of the projection surface (14) is controllably configured to display the third operating condition in a color other than the colors of the first and second operating conditions, wherein the predetermined third portion (38) is larger than the predetermined first portion (36) and the predetermined second portion (37).

8. Extracorporeal blood treatment device according to one of the claims 3 to 5, in which an at least two-channel input evaluation of an interaction of the user by means of at least a first and a second position detecting system is provided.

## Revendications

1. Appareil extracorporel de traitement du sang avec un dispositif d'affichage, comprenant:
un(e) surface ou panneau de projection (14) monté(e) au niveau de l'appareil extracorporel de traitement du sang et configuré(e) pour présenter de manière visible sur un côté avant de la surface/du panneau de projection (14) un contenu d'affichage (13) prédéterminé pour un utilisateur de l'appareil extracorporel de traitement du sang ; et
un dispositif de projection (16) monté au niveau de l'appareil extracorporel de traitement du sang et configuré pour projeter sur la surface de projection (16) le contenu d'affichage (13) prédéterminé depuis un côté arrière de la surface de projection (14),
**caractérisé en ce que**
une section prédéterminée de la surface de projection (14) destinée à l'affichage d'au moins un état de fonctionnement prédéterminé de l'appareil extracorporel de traitement du sang est prévue,
dans au moins des premier et deuxième états de fonctionnement, une première et/ou une deuxième section (36, 37) prédéterminée(s) de la surface de projection (14) est/sont configurée(s) pour pouvoir être activée(s) et afficher les premier et deuxième états de fonctionnement dans différentes couleurs,
la surface de projection est articulée au moyen d'un dispositif articulé, qui est un agencement formant articulation à la manière d'une charnière, au niveau d'une section de surface de l'appareil extracorporel de traitement du sang au niveau d'une formation ou élévation en forme de table et/ou est guidée dans un agencement formant cadre placé au moins dans le sens de la hauteur par rapport à l'appareil extracorporel de traitement du sang et peut coulisser à l'intérieur dudit agencement formant cadre,
le dispositif articulé et/ou l'agencement formant cadre sont configurés de manière à pouvoir être déplacé(s) en hauteur ensemble de telle manière que la surface de projection (14) peut être insérée au moins partiellement dans un évidement de l'appareil extracorporel de traitement du sang.

2. Appareil extracorporel de traitement du sang selon la revendication 1, dans lequel la surface de projection (14) est une surface de projection (14) translucide constituée d'un matériau semi-opaque.

3. Appareil de traitement extracorporel du sang selon l'une quelconque des revendications précédentes, comprenant en outre au moins un élément d'interaction (32, 34) agencé sur la surface de projection (14) et configuré pour détecter une interaction de l'utilisateur par sensibilité au contact.

4. Appareil extracorporel de traitement du sang selon l'une quelconque des revendications précédentes, comprenant en outre un dispositif d'imagerie et/ou un dispositif formant capteur optique, configuré pour détecter une interaction de l'utilisateur sans qu'il y ait contact.

5. Appareil extracorporel de traitement du sang selon l'une quelconque des revendications précédentes, configuré pour mettre en œuvre une reconnaissance de geste et/ou de position en 2D et/ou 3D sur un plan de projection.

6. Appareil extracorporel de traitement du sang selon l'une quelconque des revendications précédentes, dans lequel au moins un élément de commande (34) pouvant être actionné manuellement par l'utilisateur est intégré dans la surface de projection (14).

7. Appareil extracorporel de traitement du sang selon la revendication 1, dans lequel, dans un troisième état de fonctionnement, une troisième section (38) prédéterminée de la surface de projection (14) est configurée pour pouvoir être activée et afficher le troisième état de fonctionnement dans une couleur différente par rapport aux premier et deuxième états de fonctionnement, dans lequel la troisième section (38) prédéterminée est plus grande que la première section (36) prédéterminée et la deuxième section (37) prédéterminée.

8. Appareil extracorporel de traitement du sang selon l'une quelconque des revendications 3 à 5, dans lequel une évaluation d'entrée au moins bicanal d'une interaction de l'utilisateur est prévue au moyen d'au moins des premier et second systèmes de reconnaissance de position.
